# FASCICULE DE BREVET EUROPEEN

(11) **EP 3 636 307 B1**
(45) Date de publication et mention de la délivrance du brevet: **16.06.2021**
(21) Numéro de dépôt: 19199468.0
(22) Date de dépôt: 25.09.2019
(51) Int. Cl.: A61M 16/00, A61M 16/06

(54) **APPAREIL MÉDICAL DE VENTILATION AVEC SÉLECTEURS DE CATÉGORIE DE PATIENT ET DE MODES DE VENTILATION COMPATIBLES**
MEDIZINISCHE BEATMUNGSVORRICHTUNG MIT KOMPATIBLER PATIENTENKATEGORIE UND VENTILATIONSMODUSAUSWAHLEN
MEDICAL VENTILATION DEVICE WITH COMPATIBLE PATIENT CATEGORY AND VENTILATION MODE SELECTORS

(30) Priorité: 12.10.2018 FR 1859472
(43) Date de publication de la demande: 15.04.2020
(73) Titulaire: Air Liquide Medical Systems, 92160 Antony (FR)
(72) Inventeur: RUSSO, Leslie, 92160 Antony (FR); DERACHE, Maxime, 92160 Antony (FR)
(74) Mandataire: Air Liquide

(56) Documents cités:
- WO-A1-98/41270
- FR-A3- 3 051 117
- US-A- 6 099 481
- US-A1- 2008 072 902

## Description

L'invention concerne les appareils ou dispositifs de ventilation artificielle comprenant des moyens de sélection simples et intuitifs des paramètres ventilatoires que sont le mode de ventilation et la catégorie de patient à ventiler, permettant de renforcer la sécurité ventilatoire en évitant des choix de paramètres ventilatoires incompatibles les uns avec les autres.

Les dispositifs ou appareils de ventilation artificielle, aussi appelés appareils d'assistance respiratoire ou ventilatoire, ou plus simplement ventilateurs médicaux, sont utilisés pour délivrer un gaz respiratoire, tel de l'air ou de l'air enrichi en oxygène, aux patients souffrant de certaines pathologies ou troubles respiratoires, par exemple d'apnée du sommeil. Les patients peuvent être des adultes ou des enfants, voire même des nourrissons, c'est-à-dire des bébés.

En général, un ventilateur médical propose différents modes de ventilations que l'utilisateur, i.e. le personnel soignant, peut sélectionner en fonction de la pathologie du patient à soigner, par exemple un mode de ventilation dédié à la réanimation cardio-pulmonaire, dit « mode CPV », pour un patient en arrêt cardiaque, ou un mode de ventilation à pression continue, dit « mode CPAP », pour un patient souffrant d'apnée du sommeil...

L'utilisateur peut ensuite paramétrer le mode de ventilation choisi, par exemple via un réglage du ou des niveaux de pression souhaité(s), de la teneur en oxygène (FiO₂) désirée ..., ou bien sélectionner des paramètres par défaut (pré)enregistrés dans une mémoire du ventilateur. En effet, un dispositif de ventilation artificielle propose en général une pluralité de réglages par défaut pour chaque mode et pour chaque type de patient, à savoir adulte, enfant ou nourrisson, i.e. bébé ou nouveau-né.

Le choix du mode de ventilation et du type ou catégorie de patient à ventiler sont assurés grâce à un système de réglage intégré au dispositif.

Ainsi, US2008/072902 propose un appareil de ventilation artificielle comprenant une micro-soufflante, des moyens de pilotage, des moyens de mémorisation et des moyens de sélection actionnables par un utilisateur incluant un ou plusieurs sélecteurs de catégorie de patient.

Or, un mode de ventilation donné proposé par un appareil d'assistance respiratoire n'est pas forcément utilisable pour toutes les catégories, i.e. les types de patients possibles.

Par exemple, en fonction des spécifications de l'appareil, il arrive que le mode « CPV » ne soit utilisable que sur des patients adultes, du fait qu'il intègre par exemple des capteurs non adaptés aux enfants ou aux nourrissons, qui font qu'il est impossible d'assurer un mode CPV efficace sur des patients non-adultes.

Un utilisateur ne doit donc pas pouvoir se tromper en sélectionnant un mode ventilatoire non compatible avec une catégorie de patient donnée qui est non-compatible avec le mode sélectionné.

Or, ceci n'est souvent pas pris en compte dans les appareils existants et pose dès lors de sérieux problèmes de sécurité de ventilation et d'efficacité de traitement pour les patients, pouvant conduire à des barotraumatismes ou des ventilations inadaptées et peu ou pas efficaces, par exemple une hypoventilation. On risque aussi de fournir des monitorages erronés qui peuvent conduire à des erreurs de diagnostic et donc à des erreurs de réglages qui eux-mêmes peuvent déclencher une sur-insufflation de gaz, donc conduire à un risque de barotraumatisme.

Par ailleurs, lors de la mise sous tension du ventilateur, c'est-à-dire lors de sa mise en route, il est impératif que la ventilation ne débute pas avant que l'utilisateur ait explicitement sélectionné le mode de ventilation adapté à la pathologie du patient à traiter.

Le problème est dès lors de proposer un dispositif ou appareil de ventilation artificielle amélioré permettant de réaliser, après mise sous tension de l'appareil, une sélection simple et intuitive des paramètres ventilatoires que sont le mode de ventilation et la catégorie (ou type) de patient à ventiler, sans toutefois qu'il ne soit possible de sélectionner un mode de ventilation non adapté à un patient donné, et de démarrer ensuite une ventilation basée sur les paramètres sélectionnés, de préférence en maximum deux opérations, de manière à renforcer la sécurité pour les patients et à assurer une efficacité de traitement accrue.

La solution de l'invention concerne alors un appareil de ventilation artificielle, c'est-à-dire un ventilateur médical adapté à la ventilation de patients, comprenant :
- une micro-soufflante, aussi appelée compresseur ou turbine, reliée fluidiquement à un circuit de gaz pour alimenter ledit circuit de gaz, en gaz respiratoire, tel de l'air ou de l'air enrichi en oxygène,
- des moyens de pilotage commandant la micro-soufflante,
- des moyens de mémorisation configurés pour mémoriser au moins plusieurs modes ventilatoires, et
- des moyens de sélection actionnables par un utilisateur, typiquement un personnel soignant (e.g. médecin, infirmière...) pour permettre audit utilisateur, de sélectionner une catégorie de patient désirés et un mode ventilatoire,
caractérisé en ce que les moyens de sélection comprennent :
- un ou plusieurs sélecteurs de catégorie de patient permettant de sélectionner au moins une catégorie de patient donnée parmi plusieurs catégories de patient sélectionnables, et
- un ou plusieurs sélecteurs de mode de ventilation permettant de sélectionner au moins un mode de ventilation parmi plusieurs modes de ventilation sélectionnables en fonction de la catégorie de patient donnée sélectionnée au moyen dudit au moins un sélecteur de catégorie de patient,
et dans lequel le ou les sélecteurs de mode de ventilation sont configurés pour qu'au moins un mode de ventilation ne soit pas sélectionnable en association avec au moins une catégorie de patient sélectionnée par le ou les sélecteurs de catégorie de patient.

En d'autres termes, selon l'invention, le(s) sélecteur(s) de mode de ventilation et/ou le/les sélecteurs de catégorie de patient coopèrent ensemble, notamment sont adaptés et/ou conçus, pour qu'il soit impossible de sélectionner un mode de ventilation non adapté à une catégorie de patient donnée.

Une fois le sélecteur de catégorie de patient activé par l'utilisateur par action digitale ou manuelle sur le ou l'un des sélecteurs de catégorie, seuls les modes de ventilation adaptés à la catégorie de patient choisie peuvent être sélectionnés au moyen du/des sélecteur(s) de mode de ventilation. Certaines associations modes/catégories sont donc rendues impossibles ou inopérantes de sorte d'améliorer la sécurité pour le patient en évitant que le choix d'un mode erroné soit possible.

Selon le cas, l'appareil de ventilation de l'invention peut comprendre l'une ou plusieurs des caractéristiques techniques suivantes :
- le ou les sélecteurs de catégorie de patient et le ou les sélecteurs de mode de ventilation sont choisis parmi les boutons-sélecteurs rotatifs et les touches de sélection.
- de préférence, les sélecteurs de catégorie de patient comprennent plusieurs touches de sélection.
- les sélecteurs de catégorie de patient comprennent plusieurs touches de sélection actionnables par actionnement digital d'un utilisateur, c'est-à-dire typiquement par pression du doigt, par exemple de l'index.
- les sélecteurs de catégorie de patient comprennent de 2 à 10 touches de sélection, de préférence de 3 à 5 touches.
- les sélecteurs de catégorie de patient comprennent trois touches de sélection configurées pour permettre de sélectionner une catégorie de patients donnée choisie parmi les catégories (de patients) adulte, enfant et nourrisson.
- les touches de sélection de catégorie de patient sont repérées chacune par un pictogramme (i.e. représentation graphique) représentant une catégorie de patient donnée.
- les trois touches de sélection de catégorie de patient sont repérées respectivement par un premier pictogramme représentant un adulte, un deuxième pictogramme représentant un enfant et un troisième pictogramme représentant un nourrisson.
- les sélecteurs de mode de ventilation comprennent plusieurs touches de sélection actionnables par actionnement digital d'un utilisateur, c'est-à-dire typiquement par pression du doigt, par exemple de l'index.
- les sélecteurs de mode de ventilation comprennent au moins trois touches de sélection configurées pour permettre de sélectionner chacune un mode de ventilation donné parmi plusieurs modes de sélection enregistrés dans les moyens de mémorisation, typiquement entre 4 et 10 touches de sélection, de préférence entre 5 et 8 touches de sélection.
- il comprend en outre une touche de mise en pause (*stand-by* en anglais) correspondant à un mode de veille ou mode pause, dans lequel aucun mode ventilatoire n'est sélectionné. Lorsque l'appareil est en pause ou veille, les moyens de pilotage ne commandent pas de fourniture de gaz par la micro-soufflante, c'est-à-dire que la ventilation par envoi de gaz ne se fait pas lorsque cette touche de mise en pause est activée par l'utilisateur.
- les touches de sélection de mode de ventilation et/ou la touche de mise en pause sont repérées chacune par un repère lumineux, de préférence des diodes de type LED ou analogue.
- les repères lumineux sont agencés à côté, c'est-à-dire en regard et à proximité, des touches de sélection de mode de ventilation et/ou de la touche de mise en pause.
- les repères lumineux sont portés par la carcasse du ventilateur, par exemple fixés au travers de la paroi de la carcasse.
- les touches de sélection de catégorie de patient sont agencées en une première ligne ou en rangée, i.e. alignées.
- les touches de sélection de mode de ventilation sont agencées en une seconde ligne ou en rangée, i.e. alignées.
- la première ligne ou en rangée et la seconde ligne ou en rangée sont parallèles ou non.
- les touches de sélection de mode de ventilation sont identiques ou différentes les uns des autres, de préférence des touches identiques ou semblables, notamment en termes de dimensions et de formes.
- les touches de sélection de catégorie de patient sont identiques ou différentes les uns des autres, de préférence des touches identiques ou semblables, notamment en termes de dimensions et de formes.
- les touches de sélection de catégorie de patient et/ou les touches de sélection de mode de ventilation sont rétroéclairées.
- les moyens de pilotage commandant la micro-soufflante comprennent au moins un microprocesseur, en particulier au moins un microcontrôleur.
- les moyens de pilotage commandant la micro-soufflante comprennent au moins une carte électronique comprenant ledit au moins un microprocesseur.
- les moyens de pilotage commandant la micro-soufflante comprennent au moins un microprocesseur mettant en oeuvre un ou des algorithmes.
- les moyens de mémorisation comprennent une ou plusieurs mémoires.
- les moyens de mémorisation comprennent une (ou des) mémoire rapide ou RAM servant notamment à enregistrer provisoirement ou temporairement des paramètres, des données, des informations... En général, une mémoire RAM permet un enregistrement plus rapide mais qui est effacé avec l'extinction de l'appareil.
- les moyens de mémorisation comprennent une (ou des) mémoire flash servant à enregistrer des paramètres, des données, des informations... et les conserver à demeure au sein de l'appareil, notamment des modifications des paramètres des différents modes ventilatoires opérées par l'utilisateur.
- de préférence, la mémoire flash est une mémoire persistante qui conserve les données enregistrées après l'arrêt de l'appareil.
- les moyens de mémorisation sont configurés pour sauvegarder ou non les réglages de l'utilisateur lors de l'extinction volontaire de l'appareil.
- au moins une mémoire est portée par la carte électronique comprenant le (ou les) microprocesseur(s) des moyens de pilotage.
- les moyens de mémorisation sont configurés pour enregistrer (i.e. mémoriser) les modes ventilatoires associés à une ou plusieurs catégories de patients.
- les moyens de mémorisation sont configurés pour enregistrer (i.e. mémoriser) les paramètres des différents modes ventilatoires.
- les moyens de pilotage coopèrent avec les moyens de mémorisation pour retrouver le ou les modes de ventilation mémorisés correspondant à la catégorie de patient ayant été sélectionnée au moyen du ou des sélecteurs de catégorie de patient, en particulier les touches de sélection de catégorie de patient, c'est-à-dire pour retrouver le ou les modes de ventilation mémorisés en association avec une catégorie de patient donnée.
- les moyens de pilotage coopèrent avec le ou les sélecteurs de mode de ventilation, en particulier les touches de sélection de mode de ventilation, pour autoriser la sélection d'au moins un mode de ventilation par actionnement par l'utilisateur d'un ou plusieurs sélecteurs de mode de ventilation, en particulier une ou plusieurs touches de sélection de mode de ventilation. Ceci permet d'autoriser une sélection par l'utilisateur de tous les modes de ventilation adaptés à une catégorie de patient sélectionnée, par exemple d'autoriser la sélection de tous les modes de ventilation pour un patient adulte.
- les moyens de pilotage coopèrent avec le ou les sélecteurs de mode de ventilation, en particulier les touches de sélection de mode de ventilation, pour interdire ou rendre inopérant la sélection d'au moins un mode de ventilation par actionnement par l'utilisateur d'un ou plusieurs sélecteurs de mode de ventilation, en particulier une ou plusieurs touches de sélection de mode de ventilation. Ceci permet d'empêcher une sélection par l'utilisateur d'un mode de ventilation non-adapté à la catégorie de patient sélectionnée, par exemple empêcher d'adopter le mode CPV pour un nourrisson.
- les moyens de pilotage commandent au moins la micro-soufflante en fonction de la catégorie de patient et du mode de ventilation associé ayant été sélectionnés au moyen du ou des sélecteurs de catégorie de patient et du ou des sélecteurs de mode de ventilation.
- les moyens de mémorisation sont configurés pour mémoriser plusieurs modes de ventilation choisis parmi les modes CPV (ventilation cardio-pulmonaire pour réanimation cardiaque), VPAC (Ventilation à Pression Assistée Contrôlée avec déclenchement inspiratoire, i.e. *trigger* en anglais, pour un patient ayant suffisamment de force pour déclencher un cycle respiratoire. L'activation/désactivation du déclenchement fait partie des paramètres modifiables par l'utilisateur), VSAI (Ventilation Spontanée sous Aide Inspiratoire utilisée par exemple dans le cadre du sevrage du patient ou dans la prise en charge de décompensation de patient atteint de BPCO (Broncho-Pneumopathie Chronique Obstructive), CPAP (ventilation à pression continue) et HFOT (ventilation à flux élevé d'oxygène).
- les moyens de mémorisation sont configurés pour mémoriser un mode de mise en veille (pause) dans lequel aucun mode de ventilation n'est sélectionné et/ou dans lequel aucune ventilation n'est délivrée, i.e. opérée, par l'appareil.
- chaque mode de ventilation comprend des paramètres de ventilation comprenant une ou des valeurs de pression de gaz, de débit de gaz et de fréquence respiratoire, et éventuellement de FiO₂. Les paramètres de ventilation dépendent du mode de ventilation sélectionné par l'utilisateur, par exemple :
   - dans le mode CPV, l'utilisateur peut régler la consigne de FiO₂, la consigne d'aide inspiratoire appelée pression haute ou PH (i.e. une valeur de pression), la consigne de fréquence respiratoire et la consigne de PEP ou pression expiratoire positive, aussi appelée pression basse ou PB (avec PB<PH).
   - dans le mode HFOT, l'utilisateur peut régler la consigne de FiO₂ et la consigne de débit.
   - dans le mode VPAC, l'utilisateur peut régler la consigne de FiO₂, la consigne d'aide inspiratoire, la consigne de fréquence respiratoire, la consigne de PEP et la désactivation/activation du déclenchement inspiratoire (i.e. trigger).
   - dans le mode VSAI, l'utilisateur peut régler la consigne de FiO₂, la consigne d'aide inspiratoire et la consigne de PEP.
   - et dans le mode CPAP, l'utilisateur peut régler la consigne de FiO₂ et la consigne de CPAP (valeur de pression).
- les moyens de pilotage commandent au moins la micro-soufflante en fonction d'un ou plusieurs paramètres ventilatoires du mode de ventilation sélectionné correspondant à la catégorie de patient choisie.
- selon un autre mode de réalisation, le sélecteur de catégorie de patient et le sélecteur de mode de ventilation comprennent des boutons-sélecteurs rotatifs, aussi appelés commutateurs rotatifs.
- un premier bouton-sélecteur rotatif permet de choisir la catégorie de patient.
- un second bouton-sélecteur rotatif permet de choisir un mode de ventilation correspondant à la catégorie de patient sélectionnée.
- les boutons-sélecteurs rotatifs sont manœuvrables en rotation par l'utilisateur, dans le sens des aiguilles d'une montre et/ou dans le sens contraire.
- les boutons-sélecteurs rotatifs manœuvrables sont mobiles en rotation entre plusieurs positions prédéfinies correspondant à des modes de ventilation différents, c'est-à-dire que chaque position correspondant à un mode de ventilation donné dont les réglages (i.e. valeurs de consigne...) sont prédéfinis et enregistrés
- le second bouton-sélecteur rotatif est manœuvrable entre plusieurs positions prédéfinies correspondant à des modes ventilatoires et une position de veille correspondant à un mode de « pause », dans lequel la ventilation par envoi de gaz ne se fait pas, c'est-à-dire qu'aucun gaz n'est délivré par la micro-soufflante.
- les boutons-sélecteurs rotatifs sont mobiles en rotation entre plusieurs positions angulairement décalées les unes des autres, c'est-à-dire agencées décalées en en arc de cercle
- les repères lumineux sont disposés autour des boutons-sélecteurs rotatifs en regard des positions angulairement décalées, en particulier en arc de cercle.
- les noms des modes et les noms ou représentations graphiques des catégories de patients peuvent être sérigraphiés ou apposés (e.g. collés) sur la carcasse de l'appareil en regard des boutons-sélecteurs rotatifs.
- selon encore un autre mode de réalisation, aussi appelé mode de réalisation « mixte », les sélecteurs de catégorie de patient comprennent plusieurs touches de sélection (comme dans le premier mode de réalisation décrit ci-avant), alors que le sélecteur de mode de ventilation comprend un bouton-sélecteur rotatif (comme dans le second mode de réalisation décrit ci-avant), ou inversement.

D'une façon générale, quel que soit le mode de réalisation choisi, l'appareil de ventilation de l'invention comprend peut comprendre aussi l'une ou plusieurs des caractéristiques techniques suivantes :
- la micro-soufflante délivre de l'air, de l'air enrichi en oxygène ou de l'oxygène pur en tant que gaz respiratoire, de préférence de l'air.
- la micro-soufflante comprend un moteur électrique.
- la micro-soufflante comprend un moteur électrique comprenant un axe ou arbre rotatif portant au moins une roue à ailettes.
- la micro-soufflante comprend un moteur électrique surmonté d'une volute agencée autour de la roue à ailettes, c'est-à-dire que la volute définit un compartiment interne dans lequel se trouve la roue à ailettes.
- la volute comprend une entrée de gaz et une sortie de gaz.
- la volute est surmontée d'un pavillon.
- le moteur est conçu pour pouvoir atteindre une vitesse de rotation allant jusqu'à au moins 40 000 tours/minute (tr/min), préférentiellement jusqu'à 70 000 tr/min environ.
- la micro-soufflante est commandée par les moyens de pilotage.
- le moteur est de type sans balai (i.e. *brushless* en anglais).
- le moteur est de type à faible inertie.
- il comprend des moyens d'alimentation en courant électrique, tels que batterie rechargeable et/ou cordon avec prise de raccordement au secteur (i.e. 110/220 V).
- les moyens d'alimentation en courant électrique alimentent en courant électrique les différents composants nécessitant de l'électricité pour fonctionner, en particulier moteur électrique de la micro-soufflante, moyens de pilotage, notamment la carte électronique, les diodes ou autres éclairages de l'appareil, etc...
- il comprend une carcasse externe dans laquelle sont agencés la micro-soufflante, tout ou partie du circuit de gaz, les moyens de pilotage commandant la micro-soufflante, typiquement une carte électronique à microcontrôleur, et les moyens de mémorisation, en particulier la mémoire flash et la mémoire RAM.
- le ou les sélecteurs de catégorie de patient et le ou les sélecteurs de mode de ventilation sont agencés (i.e. portés) par la carcasse externe de l'appareil.
- il comprend un capteur de pression et/ou un capteur de débit agencés de manière à mesurer la pression et/ou le débit du gaz dans le circuit de gaz, en aval de la micro-soufflante.
- il comprend au moins un actionneur agencé en aval de la micro-soufflante pour contrôler le débit de gaz dans le circuit de gaz, en particulier une ou des électrovannes.
- les moyens de pilotage sont configurés pour :
   i) retrouver parmi les modes ventilatoires mémorisés par les moyens de mémorisation, au moins un mode ventilatoire donné en réponse à une sélection par l'utilisateur d'une catégorie de patient et dudit mode ventilatoire par actionnement d'une des touches de sélection de catégorie de patient et d'une des touches de sélection de mode ventilatoire,
   ii) commander au moins la micro-soufflante de sorte qu'elle délivre du gaz respiratoire selon le mode ventilatoire sélectionné.
- les moyens de pilotage sont configurés pour commander au moins la micro-soufflante et au moins un actionneur, notamment une ou plusieurs électrovannes.
- l'appareil comprend une interface graphique utilisateur ou IGU et/ou une interface homme-machine ou IHM.
- l'IGU et/ou l'IHM comprend des moyens de réglage (e.g. touches, boutons, curseurs ou autres) et un afficheur graphique, c'est-à-dire un écran d'affichage graphique, par exemple tactile avec de préférence des touches virtuelles.
- l'appareil est configuré pour qu'une catégorie de patient donnée, par exemple la catégorie adulte, soit sélectionnée par défaut, en particulier lors de la mise en route de l'appareil. Dans ce cas, une action de l'utilisateur suffit puisqu'il suffit alors de sélectionner un mode de ventilation compatible pour démarrer la ventilation en catégorie adulte.
- l'appareil comprend en outre une touche de validation permettant de confirmer la sélection d'un mode de ventilation au moyen du ou des sélecteurs de mode de ventilation.
- l'appareil est configuré pour être, par défaut, par exemple lors de sa mise en route, i.e. allumage, en catégorie de patient adulte et en mode pause.
- selon un mode de réalisation, l'appareil est configuré pour que passer du mode pause à un mode de ventilation, avec démarrage (de préférence immédiat) de la ventilation par commande de la micro-soufflante, requiert successivement l'activation par l'utilisateur au moins :
   a) du ou des sélecteurs de mode de ventilation pour sélectionner un mode de ventilation donné compatible avec la catégorie de patient sélectionnée, et
   b) de la touche de validation pour confirmer la sélection du mode de ventilation sélectionné.
      - selon un autre mode de réalisation, l'appareil est configuré pour que passer du mode pause à un mode de ventilation, avec démarrage (de préférence immédiat) de la ventilation par commande de la micro-soufflante, requiert uniquement une activation par l'utilisateur du ou des sélecteurs de mode de ventilation pour sélectionner un mode de ventilation donné compatible avec la catégorie de patient sélectionnée, c'est-à-dire sans validation par actionnement de la touche de validation.
      - l'appareil est par ailleurs configuré pour que, passer du mode pause à un mode de ventilation requiert, préalablement à la sélection d'un mode de ventilation donné, une activation par l'utilisateur du ou des sélecteurs de catégorie de patient pour sélectionner une catégorie de patient désirée, et ce, quel que soit le mode de réalisation considéré.
      - selon un autre mode de réalisation, l'appareil est par ailleurs configuré pour que, passer du mode pause à un mode de ventilation requiert uniquement une sélection d'un mode de ventilation donné, lorsqu'une catégorie patient est sélectionnée par défaut au démarrage de l'appareil ou a déjà été sélectionnée préalablement. Dans ce cas, l'utilisateur n'a pas à sélectionner la catégorie de patient par actionnement du ou des sélecteurs de catégorie de patient puisque le patient à traiter correspond à la catégorie de patient par défaut ou ayant déjà sélectionnée lors d'une précédente ventilation.
      - l'appareil est en outre configuré pour qu'au cours d'une ventilation, c'est-à-dire après démarrage de la ventilation d'un patient, deux actions successives soient requises pour modifier le mode de ventilation, à savoir d'abord le choix d'un nouveau mode de ventilation compatible avec la catégorie de patient sélectionnée, suivi d'une validation de ce nouveau mode de ventilation par activation/actionnement de la touche de validation. Ceci permet d'éviter tout changement de mode de ventilation intempestif ou par erreur.
      - il est configuré pour qu'au cours d'une ventilation, modifier le mode de ventilation requiert successivement l'activation par l'utilisateur :
         a) du ou des sélecteurs de mode de ventilation pour passer d'un premier mode de ventilation à un second mode de ventilation désiré compatible avec la catégorie de patient sélectionnée, et
         b) de la touche de validation pour confirmer la sélection dudit second mode de ventilation sélectionné.
            - en particulier, le passage ou la sélection du mode HFOT (ventilation à flux élevé d'oxygène) requiert toujours une activation par l'utilisateur de la touche de validation pour confirmer la sélection du mode HFOT. En effet, le mode HFOT est un mode qui requiert une interface respiratoire spécifique, donc il ne doit pas être possible de le lancer sans qu'une confirmation ne soit requise après sa sélection.

L'invention va maintenant être mieux comprise grâce à la description détaillée suivante, faite à titre illustratif mais non limitatif, en référence aux figures annexées parmi lesquelles :
- les Figures 1 et 2 schématisent un premier mode de réalisation d'un appareil de ventilation artificielle selon la présente invention, et
- les Figures 3 et 4 schématisent d'autres modes de réalisation d'un appareil de ventilation artificielle selon la présente invention.

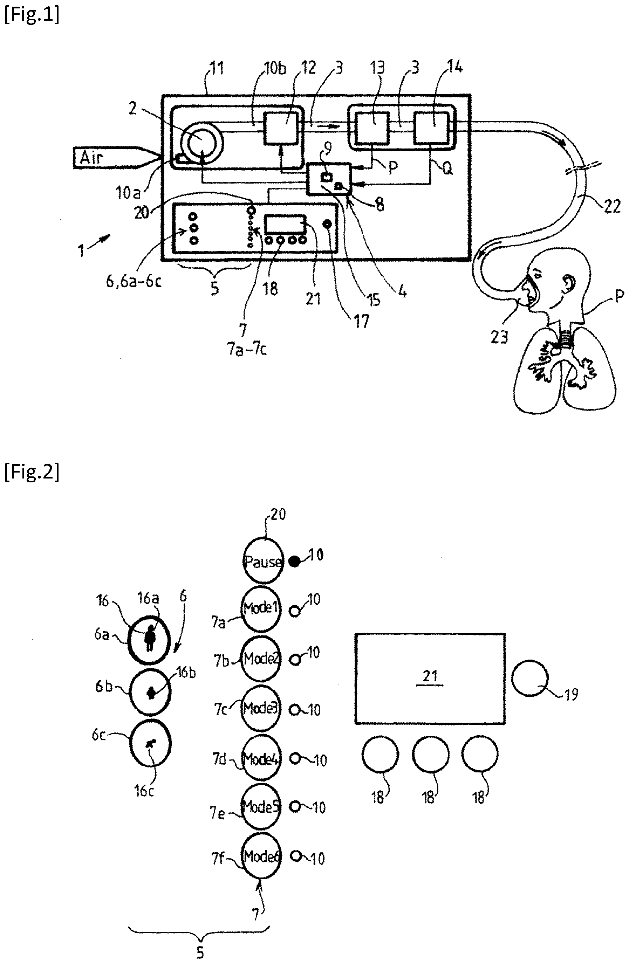

La Figure 1 est un schéma d'un mode de réalisation d'un appareil de ventilation 1 ou ventilateur médical selon la présente invention, et la Figure 2 schématise un premier mode de réalisation des moyens de sélection 5 de catégorie de patient 6 et de mode ventilatoire 7 selon l'invention, incorporés dans l'appareil de la Figure 1.

Cet appareil de ventilation 1 comprend une carcasse ou coque externe 11, par exemple en polymère, dans laquelle sont agencés les différents composants de l'appareil 1. Plus précisément, cet appareil 1 comprend une micro-soufflante 2, aussi appelée turbine ou compresseur, aspirant de l'air ambiant via une entrée d'air 10a et par ailleurs reliée fluidiquement, en aval/sortie 10b, à un circuit de gaz 3, comprenant un passage ou conduit interne de gaz, de sorte d'alimenter ce circuit de gaz 3, en gaz respiratoire délivré par la micro-soufflante 2.

Typiquement, le gaz respiratoire est de l'air, voire de l'air enrichi en oxygène ou de l'oxygène pur, notamment en fonction de la thérapie/ventilation à mettre en œuvre.

Le circuit de gaz 3 interne de l'appareil 1 est relié fluidiquement au patient P via un conduit flexible 22 et une interface respiratoire 23, tel un masque respiratoire, une sonde trachéale ou analogue, de manière à alimenter le patient P en gaz respiratoire.

La micro-soufflante 2 est motorisée, c'est-à-dire qu'elle comporte un moteur électrique, typiquement un moteur sans balais (i.e. *brushless*) ayant une vitesse de rotation pouvant atteindre 40 000 tr/min, voire 70 000 tr/min, et préférentiellement ayant aussi une faible inertie. Classiquement, une telle micro-soufflante 2 comprend un moteur électrique protégé par un carter et entraînant, pendant son fonctionnement, un axe ou arbre rotatif portant une roue à ailettes agencée dans le compartiment interne d'une volute qui surmonte le moteur électrique et le carter. La volute comprend une entrée de gaz par lequel l'air aspiré via l'entrée 10a pénètre dans le compartiment interne et une sortie de gaz par laquelle le gaz sous pression ressort du compartiment interne puis chemine au travers de la sortie 10b et dans le conduit 3. La volute peut être surmontée d'un pavillon.

Afin de contrôler le passage de gaz dans le circuit 3, c'est-à-dire en direction du patient P, il est prévu un ou plusieurs actionneurs 12, par exemple une (ou des) électrovanne(s), agencés sur le trajet du gaz (e.g. sur circuit interne), en aval de la micro-soufflante 2, ainsi qu'un ou des capteurs de pression (P) 13 et/ou de débit 14 (Q) sont également agencés sur le trajet du gaz, c'est-à-dire de manière à opérer des mesures dans le circuit de gaz 3, en particulier en aval de (ou des) l'actionneur 12.

Sont également prévus des moyens de pilotage 4 servant à commander le ou les actionneurs 12 et par ailleurs la micro-soufflante 2 en fonction de réglages mémorisés sous forme de modes ventilatoires comme expliqué ci-dessous.

Les moyens de pilotage 4 comprennent avantageusement un (ou des) microprocesseur 9, de préférence un (ou des) microcontrôleur. Typiquement, le microprocesseur 9 est porté par une carte électronique 15. Le microprocesseur 9 met en oeuvre un ou plusieurs algorithmes.

La micro-soufflante 2, le ou les actionneurs 12 ainsi que le ou les capteurs 13, 14 sont également reliés électriquement aux moyens de pilotage 4, en particulier au processeur porté par la carte électronique desdits moyens de pilotage 4. En fait, la micro-soufflante 2 est pilotée par les moyens de pilotage 4, par exemple via une valeur de tension continue générée à l'aide d'un signal tout-ou-rien, typiquement un pilotage par modulation de largeur d'impulsion (i.e. PWM pour *Pulse-Width Modulation* en anglais), ou une valeur de courant et est régulée grâce à un monitorage de la pression, notamment via les données de pression (P) mesurées par le capteur de pression 13.

Ainsi, le ou les capteurs 13, 14 de pression et/ou de débit permettent de transmettre des données de pression (P) et/ou de débit (Q) aux moyens de pilotage 4 et ces derniers peuvent agir sur le ou les actionneurs 12 notamment pour ajuster, stopper ou réguler la fourniture de gaz provenant de la micro-soufflante 2. En d'autres termes, les moyens de pilotage 4 récupèrent les signaux provenant notamment des capteurs 13, 14, les traitent/analysent, et commandent ensuite, en fonction de ce traitement, notamment le ou les actionneurs 12 et/ou la micro-soufflante 2.

Par ailleurs, il est prévu aussi des moyens de mémorisation 8, telle une ou des mémoires d'enregistrement des données, lesquels peuvent être aussi portés par la carte électronique 15. Ils sont notamment configurés pour mémoriser plusieurs modes ventilatoires et des associations catégories de patients/modes ventilatoires.

Avantageusement, l'appareil 1 comprend en tant que moyens de mémorisation 8, une mémoire rapide, i.e. mémoire flash, faisant office de mémoire morte et par ailleurs une mémoire vive ou RAM (i.e. *random* access *memory*).

La mémoire flash permet d'enregistrer et conserver le paramétrage des modes ventilatoires, c'est-à-dire les valeurs de pression, débit etc... associées à chaque mode ventilatoire, ainsi que les valeurs par défaut, les associations modes ventilatoires/catégories de patients ou d'autres données, informations, paramètres de fonctionnement ou autres... devant être conservés au sein de l'appareil 1. Les modes ventilatoires mémorisées sont par exemple un mode CPV, un mode CPAP, VPAC, VSAI, HFOT ou tout autre mode de ventilation.

Par ailleurs, la mémoire RAM permet de mémoriser temporairement des modifications éventuelles de paramètres souhaitées par l'utilisateur ou toute autre donnée, information ou paramètre provisoire. Ces modifications peuvent être entrées dans l'appareil 1 au moyen, par exemple, d'une interface graphique utilisateur (IGU) ou une interface homme-machine (IHM) comprenant des moyens de réglage 18 (e.g., boutons, touches, curseurs ou autres) et un écran d'affichage graphique 21 en couleurs ou en noir et blanc. L'écran d'affichage graphique 21 peut être un écran tactile et les moyens de réglage 18 de l'IGU ou de l'IHM servant modifier ou ajuster des réglages ventilatoires (i.e. paramètres) sont alors préférentiellement des touches virtuelles ou analogues s'affichant sur ledit écran tactile.

Les moyens de pilotage 4, en particulier le microprocesseur 9, coopèrent avec les moyens de mémorisation 8, i.e. mémoire flash et RAM, pour venir y retrouver les informations, paramètres ou autres données utiles, en particulier les associations modes ventilatoires/catégories de patients autorisées ou possibles, comme expliqué ci-après.

L'appareil 1 comprend par ailleurs des moyens de sélection 5, par exemple agencés sur l'une des parois ou faces externes de la carcasse 11, par exemple en façade, lesquels sont actionnables, par action digitale ou manuelle d'un utilisateur, c'est-à-dire un personnel soignant, tel un médecin, une infirmière ou analogue, pour lui permettre de sélectionner un mode ventilatoire donné parmi ceux mémorisés, i.e. enregistrés, par les moyens de mémorisation 5 en fonction d'une catégorie de patient désirée.

Pour ce faire, l'utilisateur sélectionne d'abord la catégorie de patient correspondant au patient P qui doit être soigné par administration de gaz respiratoire comme expliqué ci-dessous. Les catégories de patients sélectionnables comprennent de préférence les catégories adultes, enfants et nourrissons (i.e., bébés, nouveau-nés...), que ce soit des hommes ou des femmes.

En général, on entend une catégorie :
- adulte : pour les individus (i.e. environ >18 ans) ayant un volume pulmonaire de l'ordre de 200 mL à 1000 mL,
- enfant : pour les individus jeunes (i.e. environ <18 ans) ayant un volume pulmonaire de l'ordre de 50 à 300 mL, c'est-à-dire un poids de 8 kg minimum.
- nourrisson : pour les individus très jeunes ayant un volume pulmonaire de l'ordre de 20 à 70 mL, c'est-à-dire un poids de moins de 8 kg environ.

Les volumes indiqués sont davantage liés au poids du patient qu'à son âge et/ou à sa taille.

Selon l'invention, dans le mode de réalisation des Figures 1 et 2, les moyens de sélection 5 comprennent plusieurs sélecteurs de catégorie de patient 6, à savoir trois touches 6a-6c, correspondant chacun à une catégorie de patient donnée, c'est-à-dire adulte, enfant ou nourrisson, et plusieurs sélecteurs de mode de ventilation 7, à savoir six touches 7a-7f, permettant par ailleurs de sélectionner un mode de ventilation donné parmi un ou plusieurs modes de ventilation possibles, par exemple CPV, CPAP...., en fonction de la catégorie de patient donnée sélectionnée au moyen des sélecteurs de catégorie de patient 6.

Le ou les modes de ventilation non-adaptés, i.e. interdits, à une catégorie de patient donnée ne peuvent être sélectionnés au moyen des sélecteurs de mode de ventilation 7 car de telles sélections sont rendues inopérantes ou impossibles par les moyens de pilotage 4. Par exemple, dans le cas de boutons, l'utilisateur peut toujours appuyer sur un bouton correspondant à un mode de ventilation non-adapté mais l'algorithme (i.e. le logiciel) de l'appareil n'appliquera pas le nouveau mode de ventilation car il est incompatible avec la catégorie de patient choisie. Le voyant de sélection ne sera donc pas allumé.

En d'autres termes, les sélecteurs de mode de ventilation 7 permettent de sélectionner uniquement le ou les modes de ventilation appropriés au traitement de la catégorie de patient ayant été choisies par actionnement de l'un des sélecteurs 6a-6c de catégorie de patient 6. Ceci permet d'empêcher de sélectionner un mode ventilatoire non compatible avec la catégorie de patient à traiter, lorsque celle-ci est non-compatible avec ce mode ventilatoire.

Par exemple, grâce à la présente invention, le personnel soignant ne pourrait pas sélectionner le mode CPV pour traiter un nourrisson même si celui-ci est victime d'un arrêt cardiaque, car le mode CPV est un mode ventilatoire dédié aux adultes ou à certains enfants (i.e. adolescents par exemple) qui n'est pas du tout adapté aux nourrissons, donc potentiellement dangereux pour eux, du fait notamment des volumes et/ou pressions ventilatoires mis en jeu, mais aussi à cause des monitorages erronés susmentionnés.

Plus précisément, les différents modes de ventilation compatibles avec une catégorie de patient donnée, c'est-à-dire leurs paramètres ventilatoires, sont mémorisées dans la mémoire flash de la carte électronique faisant partie des moyens de mémorisation 8, de préférence ils sont mémorisés en association avec une ou des catégories de patients possibles, i.e. autorisés. Les moyens de pilotage 4, en particulier le microprocesseur 9 et son (ou ses) algorithme (i.e. logiciel), vont alors retrouver et proposer à l'utilisateur le ou les modes de ventilation possibles ayant été mémorisés en association avec la catégorie de patient sélectionnée par activation d'une touche de sélection de catégorie de patient, en autorisant alors une sélection par l'utilisateur d'une ou plusieurs touches de sélection de mode de ventilation 7a-7f et éventuellement en interdisant la sélection d'une ou plusieurs autres touches 7a-7f.

En d'autres termes, si un mode de ventilation est sélectionné par actionnement par l'utilisateur touches de sélection de mode de ventilation 7a-7f et que ce mode n'est pas compatible avec la catégorie de patient choisie, alors le logiciel n'appliquera pas les paramètres de ventilation correspondant à ce mode « interdit ». Dans ce cas, le voyant de sélection de mode 10 restera sur le mode précédent ou sur « Pause » P.

La solution de l'invention renforce la sécurité pour le patient en évitant que le personnel ne puisse se tromper lorsqu'il opte pour un mode ventilatoire et ce, en empêchant, excluant ou inactivant certaines associations mode ventilatoire/catégorie de patient non souhaitables, c'est-à-dire non compatibles car susceptibles de conduire à une remise en cause de la sécurité du patient ou à un traitement pas ou peu efficace du fait de paramètres ventilatoires pas ou mal adaptés.

Dans le mode de réalisation des Figures 1 et 2, les sélecteurs de catégorie de patient 6 et de mode de ventilation 7 comprennent des touches 6a-6c, 7a-7f à actionnement digital, c'est-à-dire par pression digitale de l'utilisateur, qui sont agencées en deux rangées de touches 6, 6a-6c; 7, 7a-7f parallèles, portées par l'une des parois de la carcasse 11 de l'appareil 1. Un aménagement différent est bien entendu possible.

Comme schématisé en Fig. 1, chaque touche de sélection de catégorie de patient 6a-6c porte un pictogramme 16 représentant une catégorie de patient donnée, à savoir ici trois touches 6a-6c portant des pictogrammes 16 représentant un adulte 16a, un enfant 16b et un nourrisson 16c, respectivement. Avantageusement, le système de sélection de catégorie de patient 6 possède autant de touches de sélection 6a-6c que de types de patients devant être supportés par l'appareil 1, c'est-à-dire pouvant être traités, c'est-à-dire que l'on peut donc prévoir moins de trois touches ou plus de trois touches selon le mode de réalisation désiré.

Plus précisément, le système de réglage, c'est-à-dire les moyens de sélection 5 actionnables par l'utilisateur pour lui permettre de sélectionner une association mode ventilatoire et catégorie de patient, est relié à au processeur présent sur la carte électronique des moyens de pilotage 4.

Lorsque l'utilisateur appuie sur l'une des touches 6a-6c de sélection de catégorie de patient, celle-ci va alors être éclairée ou rétroéclairée par des moyens d'éclairage, telle une (ou des) diode de type LED, qui s'activent lorsque l'utilisateur appuie sur la touche considérée. Par exemple, les moyens d'éclairage peuvent être agencés sous les touches 6a-6c et ces dernières peuvent être réalisées en matériau translucide de sorte de se rétroéclairer lorsqu'elles sont activées.

Par ailleurs, les sélecteurs de mode de ventilation 7 comprennent plusieurs touches de sélection 7a-7f, de préférence entre 5 et 10 touches de sélection, par exemple 6 touches ici, qui sont configurées pour permettre de sélectionner chacune un mode de ventilation donné parmi plusieurs modes de sélection, appelés Modes 1 à 6, enregistrés dans les moyens de mémorisation 8 en association avec une ou plusieurs catégories de patients.

Là encore, des moyens d'éclairage peuvent être agencés sous les touches 7a-7f et ces dernières peuvent être réalisées en matériau translucide de sorte de se rétroéclairer lorsqu'elles sont activées.

Les touches de sélection de mode de ventilation 7a-7f sont repérées chacune par un repère lumineux 10, de préférence une diode de type LED ou analogues, par exemple une diode de couleur rouge, verte, bleue, orange, blanche, jaune... Chaque repère 10 est agencé en regard d'une des touches de sélection de mode de ventilation 7a-7f de manière à indiquer, lorsqu'il s'allume, la touche 7a-7f qui est active. Ceci permet à l'utilisateur de voir immédiatement quel est le mode ventilatoire sélectionné pour la catégorie de patient choisie.

Bien entendu, on pourrait aussi, selon un autre mode de réalisation, prévoir des repères lumineux pour repérer les trois touches de sélection de catégorie de patient 6a-6c. Toutefois, un simple rétroéclairage de la touche active 6a-6c paraît suffisant en pratique.

Avantageusement, l'appareil 1 comprend aussi une touche de validation 19 permettant de confirmer la sélection d'un mode de ventilation au moyen du ou des sélecteurs de mode de ventilation 7a-7f. Plus précisément, l'appareil 1 est configuré pour qu'au cours d'une ventilation, c'est-à-dire après démarrage de la ventilation d'un patient, deux actions successives soient requises pour modifier le mode de ventilation, à savoir pour passer d'un mode ventilatoire à un autre mode ventilatoire, de sorte de minimiser le risque d'erreur ou de fausse manipulation. Ainsi, il faut d'abord effectuer un choix d'un nouveau mode de ventilation compatible avec la catégorie de patient ayant été déjà sélectionnée, par activation d'une des touches 7a-7f de sélection de mode de ventilation pour sélectionner un nouveau mode de ventilation différent du mode précédemment choisi, puis opérer une validation de ce nouveau mode de ventilation par activation/actionnement de la touche de validation 19.

Par ailleurs, l'appareil 1 peut aussi être configuré pour que l'activation de la touche de validation 19 soit obligatoire pour confirmer la sélection d'un mode de ventilation particulier, notamment d'un mode dit « à risques », typiquement lors du passage du mode pause à ce mode ventilatoire particulier, par exemple le mode HFOT qui requiert l'utilisation d'une interface respiratoire spécifique pour délivrer le gaz.

De préférence, l'appareil 1 est configuré pour qu'une catégorie de patient donnée, par exemple la catégorie adulte, soit sélectionnée par défaut, en particulier lors de la mise en route de l'appareil 1. Dit autrement, lors du démarrage de l'appareil 1, la touche 6a est sélectionnée par défaut et est rétroéclairée pour indiquer à l'utilisateur que ce choix de catégorie adulte est réalisé par défaut.

Dans ce cas, l'utilisateur peut alors :
- soit immédiatement choisir un mode ventilatoire en appuyant sur l'une des touches 7a-7f de mode de ventilation autorisés pour les patients adultes. Une action de l'utilisateur suffit alors pour sélectionner un mode de ventilation compatible et démarrer la ventilation en catégorie adulte.
- soit choisir d'abord une autre catégorie de patient, c'est-à-dire enfant ou nourrisson, en appuyant sur l'une des touches 6b, 6c de sélection de catégorie de patient, puis de sélectionner un mode de ventilation compatible en appuyant sur l'une des touches 7a-7f de mode de ventilation autorisées pour la catégorie de patient choisie, et enfin de valider ce choix par action sur la touche de validation 19, comme expliqué ci-avant.

Il est à noter que, lors du démarrage de l'appareil 1, c'est-à-dire que lorsque la touche 6a de catégorie adulte est sélectionnée par défaut, aucun mode de ventilation n'est sélectionné par défaut mais l'appareil 1 est en pause, c'est-à-dire qu'une touche de pause 20 est activée par défaut.

En effet, comme illustré en Figure 2, il est prévu aussi une touche de mise en pause 20 (*stand-by* en anglais) correspondant à un mode de veille, dans lequel aucun mode de ventilation n'est sélectionné. Cette touche pause 20 peut aussi être repéré par un repère lumineux 10, telle une diode de type LED ou analogue.

L'allumage et l'extinction des repères lumineux 10 sont commandés également par les moyens de pilotage 4.

En pause, les moyens de pilotage 4 ne commandent pas de fourniture de gaz par la micro-soufflante 2, c'est-à-dire que la ventilation par envoi de gaz ne se fait pas lorsque cette touche de mise en pause est activée par l'utilisateur. La touche pause 20 peut aussi être activée par l'utilisateur pour stopper la ventilation en désélectionnant tout mode ventilatoire. De préférence, tout arrêt de la ventilation par activation (i.e. appui sur) de la touche pause, nécessite une confirmation/validation de l'utilisateur via la touche de validation 19 pour éviter tout arrêt intempestif de la ventilation du patient.

Bien entendu, l'appareil comprend aussi des moyens d'alimentation électrique (non montrés), notamment batterie rechargeable et/ou cordon et prise de raccordement au secteur (e.g. 110/220V).

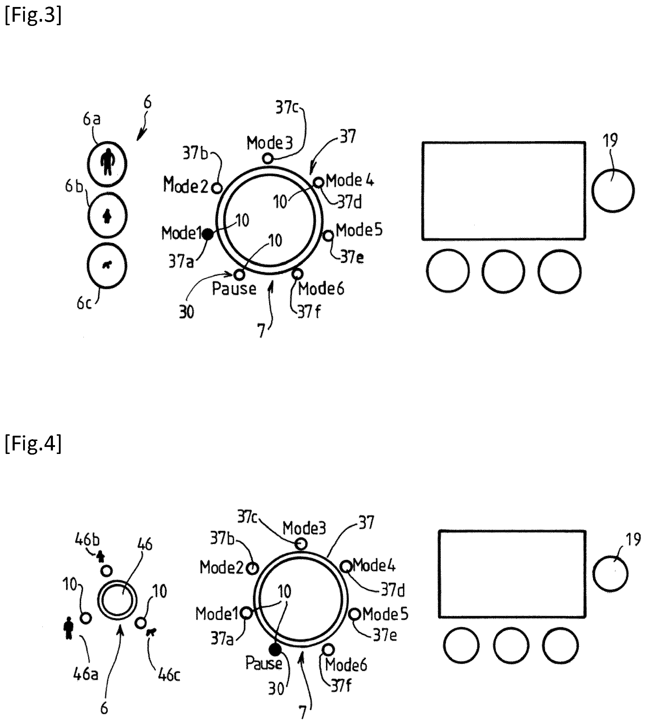

La Figure 3 représente un deuxième mode de réalisation, analogue au premier mode de réalisation des Figures 1 et 2, mais dans lequel les touches de sélection du mode de ventilation ont été remplacées par un sélecteur rotatif mécanique 37, avantageusement de type bouton-sélecteur rotatif, se déplaçant entre plusieurs positions 37a-37f, 30 angulairement décalées les unes des autres.

Chaque position angulaire 37a-37f correspond à un mode de ventilation donné, appelés respectivement « Mode 1 » à « Mode 6 », comme précédemment, par exemple ici 6 modes ventilatoires différents. Bien entendu, le nombre de modes peut être supérieur ou inférieur à 6, typiquement entre 4 et 15 modes, en général entre 5 et 12 modes. On prévoit aussi une position angulaire 30 correspondant à un mode pause ou de veille, assurant la même fonction que le bouton de pause 20 des Figures 1 et 2.

Les différentes positions 37a-37f, 30 sont agencées en (arc de) cercle autour du bouton 37 et décalées angulairement les unes des autres d'un angle égal, par exemple de l'ordre de 51°. Préférentiellement, afin d'assister l'utilisateur dans son choix de mode, les différentes positions peuvent, là encore, être repérées chacune par un repère lumineux 10, tel une LED ou analogue. Les différents repères sont disposés, également (en arc de) cercle, autour du bouton rotatif 37 en regard des positions 37a-37f des « Modes 1 à 6 » et de celle de pause 30.

Lors du fonctionnement de l'appareil 1, une fois que l'utilisateur a sélectionné la catégorie de patient en appuyant sur l'une des touches 6a-6c, comme précédemment, alors la touche sélectionnée 6a-6c est rétroéclairée et, par ailleurs, tout ou partie des différentes positions 37a-37f correspondant aux Modes 1 à 6 de ventilation, qui sont disponibles et compatibles avec la sélection de catégorie de patient opérée, s'éclairent eux aussi autour du bouton rotatif 37 de sélection de mode, alors que ceux non-disponibles et/ou non-compatibles restent éteints. Ceux-ci peut résulter, là encore, de la mise en œuvre d'un rétro-éclairage produit par des moyens d'éclairage, comme décrit ci-avant. Par défaut, le bouton rotatif 37 est en position de Pause 30.

Le bouton rotatif 37 ou sélecteur mécanique peut alors être manœuvré en rotation par l'utilisateur. La rotation du bouton 17 entraine le déplacement d'un voyant de sélection, qui passe d'un repère lumineux 10 à un autre, c'est-à-dire d'une position (37a-37f) à la position angulairement décalée suivante qui est disponible et, donc aussi d'un mode de ventilation au mode suivant, comme précédemment. Le déplacement du voyant de sélection se fait donc dans le même sens de rotation que celui du bouton rotatif 37, c'est-à-dire horaire ou antihoraire en fonction du sens dans lequel l'utilisateur tourne le bouton 37.

Le voyant de sélection « saute » les modes qui ne sont pas éclairés, empêchant ainsi toute sélection d'un mode non disponible dans la catégorie de patient sélectionnée.

Ensuite, le ventilateur 1 applique à la ventilation des réglages ou paramètres ventilatoires par défaut mémorisés et adaptés à la catégorie patient et au mode sélectionnés par l'utilisateur, comme déjà expliqué.

De préférence, la position de veille 30 correspondant à un mode de pause, aussi appelé mode « d'attente », « de veille » ou « de stand-by », dans lequel la ventilation par envoi de gaz ne se fait pas. Par défaut, il s'agit du mode qui se sélectionne automatiquement lors de l'allumage, i.e. mise en route, de l'appareil 1, en l'absence de toute action de l'utilisateur. Un repère lumineux 10 (LED) s'allumera automatiquement lors de la mise en route de l'appareil 1 pour indiquer à l'utilisateur que l'appareil est en veille.

La Figure 4 représente un troisième mode de réalisation, analogue au deuxième mode de réalisation de la Figure 3, dans lequel la sélection du mode de ventilation se fait via un premier sélecteur rotatif mécanique 37, par exemple un bouton-sélecteur rotatif, se déplaçant entre plusieurs positions 37a-37f, et une position de pause 30, qui sont angulairement décalées les unes des autres, comme déjà expliqué, et dans lequel les touches de catégorie de patient (adulte, enfant, nourrisson) ont été remplacées, elle aussi, par un second sélecteur rotatif mécanique 46, avantageusement de type bouton-sélecteur rotatif 46, se déplaçant entre plusieurs positions 46a-46c angulairement décalées les unes des autres, à savoir trois positions correspondant aux patients de types adulte, enfant et nourrisson.

Comme dans le mode de réalisation de la Figure 3, les positions des deux boutons-sélecteurs rotatifs 37, 46 sont préférentiellement indiquées par des repères lumineux 10, telles des diodes qui s'éclairent.

## Revendications

1. Appareil de ventilation (1) comprenant :
- une micro-soufflante (2) reliée fluidiquement à un circuit de gaz (3) pour alimenter ledit circuit de gaz (3), en gaz respiratoire,
- des moyens de pilotage (4) commandant la micro-soufflante (2),
- des moyens de mémorisation (8) configurés pour mémoriser au moins plusieurs modes ventilatoires (Modes 1-6), et
- des moyens de sélection (5) actionnables par un utilisateur pour permettre audit utilisateur, de sélectionner un mode ventilatoire et une catégorie de patient désirés, **caractérisé en ce que** les moyens de sélection (5) comprennent :
- un ou plusieurs sélecteurs de catégorie de patient (6) permettant de sélectionner au moins une catégorie de patient donnée parmi plusieurs catégories de patient (6) sélectionnables, et
- un ou plusieurs sélecteurs de mode de ventilation (7) permettant de sélectionner au moins un mode de ventilation parmi plusieurs modes de ventilation (Modes 1-6) sélectionnables en fonction de la catégorie de patient donnée sélectionnée au moyen dudit au moins un sélecteur de catégorie de patient (6),
et dans lequel le ou les sélecteurs de mode de ventilation (7) sont configurés pour qu'au moins un mode de ventilation (Modes 1-6) ne soit pas sélectionnable en association avec au moins une catégorie de patient sélectionnée par le ou les sélecteurs de catégorie de patient (6).

2. Appareil selon la revendication 1, **caractérisé en ce que** le ou les sélecteurs de catégorie de patient (6) et le ou les sélecteurs de mode de ventilation (7) sont choisis parmi les boutons rotatifs et les touches de sélection.

3. Appareil selon l'une des revendications précédentes, **caractérisé en ce que** les sélecteurs de catégorie de patient (6) et les sélecteurs de mode de ventilation (7) comprennent plusieurs touches de sélection (6a-6c ; 7a-7f) actionnables par actionnement digital (i.e. pression du doigt) d'un utilisateur.

4. Appareil selon l'une des revendications précédentes, **caractérisé en ce que** les sélecteurs de catégorie de patient (6) comprennent trois touches de sélection (6a-6c) configurées pour permettre de sélectionner une catégorie de patients donnée choisie parmi les catégories adulte, enfant et nourrisson.

5. Appareil selon l'une des revendications précédentes, **caractérisé en ce que** les sélecteurs de mode de ventilation (7) comprennent au moins trois touches de sélection (7a-7f) configurées pour permettre de sélectionner chacune un mode de ventilation donné parmi plusieurs modes de sélection (Modes 1-6) enregistrés dans les moyens de mémorisation (8), de préférence entre 5 et 10 touches de sélection.

6. Appareil selon l'une des revendications précédentes, **caractérisé en ce qu'**il comprend en outre une touche de mise en pause (P) correspondant à un mode pause pour lequel aucun mode de ventilation n'est sélectionné et/ou aucune ventilation n'est opérée.

7. Appareil selon l'une des revendications précédentes, **caractérisé en ce que** les touches de sélection de mode de ventilation (7a-7f) et/ou la touche de mise en pause (P) sont repérées chacune par un repère lumineux (10), de préférence des LED.

8. Appareil selon l'une des revendications précédentes, **caractérisé en ce que** les touches de sélection (6a-6c) sont repérées chacune par un pictogramme (16) représentant une catégorie de patient donnée, en particulier un pictogramme (16) représentant un adulte (16a), un enfant (16b) et un nourrisson (16c).

9. Appareil selon l'une des revendications précédentes, **caractérisé en ce qu'**il comprend en outre une touche de validation (19) permettant de confirmer la sélection d'un mode de ventilation (Mode 1-6).

10. Appareil selon l'une des revendications précédentes, **caractérisé en ce que** les moyens de pilotage (4) coopèrent avec les moyens de mémorisation (8) pour retrouver le ou les modes de ventilation (Mode 1-6) mémorisés correspondant à la catégorie de patient ayant été sélectionnée au moyen du ou des sélecteurs de catégorie de patient (6).

11. Appareil selon l'une des revendications précédentes, **caractérisé en ce que** les moyens de pilotage (4) coopèrent avec le ou les sélecteurs de mode de ventilation (7) pour :
a) autoriser la sélection d'au moins un mode de ventilation (Mode 1-6) par actionnement par l'utilisateur d'un ou plusieurs sélecteurs de mode de ventilation (7), et/ou
b) interdire ou rendre inopérant la sélection d'au moins un mode de ventilation (Mode 1-6) par actionnement par l'utilisateur d'un ou plusieurs sélecteurs de mode de ventilation (7).

12. Appareil selon l'une des revendications précédentes, **caractérisé en ce que** les moyens de pilotage (4) commandent au moins la micro-soufflante (2) en fonction de la catégorie de patient et du mode de ventilation associé ayant été sélectionnés au moyen du ou des sélecteurs de catégorie de patient (6) et du ou des sélecteurs de mode de ventilation (7).

13. Appareil selon l'une des revendications précédentes, **caractérisé en ce que** les moyens de pilotage (4) comprennent au moins un microprocesseur (9).

14. Appareil selon l'une des revendications précédentes, **caractérisé en ce qu'**il est configuré pour être, par défaut, en catégorie de patient adulte et en mode pause.

15. Appareil selon l'une des revendications précédentes, **caractérisé en ce qu'**il est configuré pour qu'au cours d'une ventilation, modifier le mode de ventilation requiert successivement l'activation par l'utilisateur :
a) du ou des sélecteurs de mode de ventilation (7) pour passer d'un premier mode de ventilation (Mode 1-6) à un second mode de ventilation (Mode 1-6) désiré compatible avec la catégorie de patient sélectionnée, et
b) de la touche de validation (19) pour confirmer la sélection dudit second mode de ventilation sélectionné.

## Patentansprüche

1. Beatmungsvorrichtung (1), welche umfasst:
- ein Mikrogebläse (2), das mit einem Gaskreislauf (3) fluidisch verbunden ist, um den Gaskreislauf (3) mit Atemgas zu versorgen,
- Steuerungsmittel (4), die das Mikrogebläse (2) steuern,
- Speichermittel (8), die dafür ausgelegt sind, wenigstens mehrere Beatmungsmodi (Modi 1-6) zu speichern, und
- Auswahlmittel (5), die von einem Benutzer betätigbar sind, um dem Benutzer zu ermöglichen, einen gewünschten Beatmungsmodus und eine gewünschte Patientenkategorie auszuwählen,
**dadurch gekennzeichnet, dass** die Auswahlmittel (5) umfassen:
- einen oder mehrere Patientenkategoriewähler (6), welche ermöglichen, wenigstens eine gegebene Patientenkategorie aus mehreren auswählbaren Patientenkategorien (6) auszuwählen, und
- einen oder mehrere Beatmungsmoduswähler (7), welche ermöglichen, wenigstens einen Beatmungsmodus aus mehreren auswählbaren Beatmungsmodi (Modi 1-6) in Abhängigkeit von der gegebenen Patientenkategorie, die mittels des wenigstens einen Patientenkategoriewählers (6) ausgewählt wurde, auszuwählen,
und wobei der oder die Beatmungsmoduswähler (7) dafür ausgelegt sind, dass wenigstens ein Beatmungsmodus (Modi 1-6) in Verbindung mit wenigstens einer durch den oder die Patientenkategoriewähler (6) ausgewählten Patientenkategorie nicht auswählbar ist.

2. Vorrichtung nach Anspruch 1, **dadurch gekennzeichnet, dass** der oder die Patientenkategoriewähler (6) und der oder die Beatmungsmoduswähler (7) aus Drehknöpfen und Auswahltasten gewählt sind.

3. Vorrichtung nach einem der vorhergehenden Ansprüche, **dadurch gekennzeichnet, dass** die Patientenkategoriewähler (6) und die Beatmungsmoduswähler (7) mehrere Auswahltasten (6a-6c; 7a-7f) umfassen, die durch Fingerbetätigung (d. h. Druck des Fingers) eines Benutzers betätigbar sind.

4. Vorrichtung nach einem der vorhergehenden Ansprüche, **dadurch gekennzeichnet, dass** die Patientenkategoriewähler (6) drei Auswahltasten (6a-6c) umfassen, die dafür ausgelegt sind zu ermöglichen, eine gegebene Patientenkategorie auszuwählen, die aus den Kategorien Erwachsener, Kind und Säugling gewählt ist.

5. Vorrichtung nach einem der vorhergehenden Ansprüche, **dadurch gekennzeichnet, dass** die Beatmungsmoduswähler (7) wenigstens drei Auswahltasten (7a-7f) umfassen, die dafür ausgelegt sind zu ermöglichen, jeweils einen gegebenen Beatmungsmodus aus mehreren Auswahlmodi (Modi 1-6) auszuwählen, die in den Speichermitteln (8) gespeichert sind, vorzugsweise zwischen 5 und 10 Auswahltasten.

6. Vorrichtung nach einem der vorhergehenden Ansprüche, **dadurch gekennzeichnet, dass** sie außerdem eine Pausentaste (P) umfasst, die einem Pausenmodus entspricht, für den kein Beatmungsmodus ausgewählt ist und/oder keine Beatmung durchgeführt wird.

7. Vorrichtung nach einem der vorhergehenden Ansprüche, **dadurch gekennzeichnet, dass** die Auswahltasten für den Beatmungsmodus (7a-7f) und/oder die Pausentaste (P) jeweils mit einer Leuchtmarkierung (10) markiert sind, vorzugsweise mit LED.

8. Vorrichtung nach einem der vorhergehenden Ansprüche, **dadurch gekennzeichnet, dass** die Auswahltasten (6a-6c) jeweils mit einem Piktogramm (16) markiert sind, das eine gegebene Patientenkategorie darstellt, insbesondere mit einem Piktogramm (16), das einen Erwachsenen (16a), ein Kind (16b) und einen Säugling (16c) darstellt.

9. Vorrichtung nach einem der vorhergehenden Ansprüche, **dadurch gekennzeichnet, dass** sie außerdem eine Bestätigungstaste (19) umfasst, welche ermöglicht, die Auswahl eines Beatmungsmodus (Modi 1-6) zu bestätigen.

10. Vorrichtung nach einem der vorhergehenden Ansprüche, **dadurch gekennzeichnet, dass** die Steuerungsmittel (4) mit den Speichermitteln (8) zusammenwirken, um den oder die gespeicherten Beatmungsmodi (Modus 1-6) abzurufen, die der Patientenkategorie entsprechen, die mittels des oder der Patientenkategoriewähler (6) ausgewählt wurde.

11. Vorrichtung nach einem der vorhergehenden Ansprüche, **dadurch gekennzeichnet, dass** die Steuerungsmittel (4) mit dem oder den Beatmungsmoduswählern (7) zusammenwirken, um:
a) die Auswahl wenigstens eines Beatmungsmodus (Modus 1-6) durch Betätigung eines oder mehrerer Beatmungsmoduswähler (7) durch den Benutzer zu autorisieren,
und/oder
b) die Auswahl wenigstens eines Beatmungsmodus (Modus 1-6) durch Betätigung eines oder mehrerer Beatmungsmoduswähler (7) durch den Benutzer zu verhindern oder unwirksam zu machen.

12. Vorrichtung nach einem der vorhergehenden Ansprüche, **dadurch gekennzeichnet, dass** die Steuerungsmittel (4) wenigstens das Mikrogebläse (2) in Abhängigkeit von der Patientenkategorie und von dem zugehörigen Beatmungsmodus steuern, die mittels des oder der Patientenkategoriewähler (6) und des oder der Beatmungsmoduswähler (7) ausgewählt wurden.

13. Vorrichtung nach einem der vorhergehenden Ansprüche, **dadurch gekennzeichnet, dass** die Steuerungsmittel (4) wenigstens einen Mikroprozessor (9) umfassen.

14. Vorrichtung nach einem der vorhergehenden Ansprüche, **dadurch gekennzeichnet, dass** sie dafür ausgelegt ist, standardmäßig auf die Patientenkategorie "Erwachsener" und den Pausenmodus eingestellt zu sein.

15. Vorrichtung nach einem der vorhergehenden Ansprüche, **dadurch gekennzeichnet, dass** sie dafür ausgelegt ist, dass es, um während einer Beatmung den Beatmungsmodus zu ändern, erforderlich ist, dass der Benutzer nacheinander aktiviert:
a) den oder die Beatmungsmoduswähler (7), um von einem ersten Beatmungsmodus (Modus 1-6) zu einem gewünschten zweiten Beatmungsmodus (Modus 1-6) zu wechseln, der mit der ausgewählten Patientenkategorie kompatibel ist, und
b) die Bestätigungstaste (19), um die Auswahl des ausgewählten zweiten Beatmungsmodus zu bestätigen.

## Claims

1. Ventilation apparatus (1) comprising:
- a micro-blower (2) connected fluidically to a gas circuit (3) in order to supply said gas circuit (3) with respiratory gas,
- control means (4) controlling the micro-blower (2),
- storage means (8) configured to store at least several ventilation modes (Modes 1-6), and
- selection means (5) that can be actuated by a user in order to allow said user to select a desired ventilation mode and a desired patient category,
**characterized in that** the selection means (5) comprise:
- one or more patient category selectors (6) making it possible to select at least one given patient category from several selectable patient categories (6), and
- one or more ventilation mode selectors (7) making it possible to select at least one ventilation mode from among several selectable ventilation modes (Modes 1-6) depending on the given patient category selected by means of said at least one patient category selector (6),
and in which the one or more ventilation mode selectors (7) are configured such that at least one ventilation mode (Modes 1-6) is not selectable in combination with at least one patient category selected by the one or more patient category selectors (6).

2. Apparatus according to Claim 1, **characterized in that** the one or more patient category selectors (6) and the one or more ventilation mode selectors (7) are chosen from among rotary knobs and selection buttons.

3. Apparatus according to either of the preceding claims, **characterized in that** the patient category selectors (6) and the ventilation mode selectors (7) comprise several selection buttons (6a-6c; 7a-7f) that can be actuated by digital actuation (i.e. pressure of the finger) by a user.

4. Apparatus according to one of the preceding claims, **characterized in that** the patient category selectors (6) comprise three selection buttons (6a-6c) configured to permit the selection of a given patient category chosen from the categories adult, child and infant.

5. Apparatus according to one of the preceding claims, **characterized in that** the ventilation mode selectors (7) comprise at least three selection buttons (7a-7f), each configured to permit the selection of a given ventilation mode from among several selection modes (Modes 1-6) registered in the storage means (8), preferably between 5 and 10 selection buttons.

6. Apparatus according to one of the preceding claims, **characterized in that** it additionally comprises a stand-by button (P) corresponding to a stand-by mode for which no ventilation mode is selected and/or no ventilation is performed.

7. Apparatus according to one of the preceding claims, **characterized in that** the ventilation mode selection buttons (7a-7f) and/or the stand-by button (P) are each indicated by a luminous indicator (10), preferably LEDs.

8. Apparatus according to one of the preceding claims, **characterized in that** the selection buttons (6a-6c) are each indicated by a pictogram (16) depicting a given patient category, in particular a pictogram (16) depicting an adult (16a), a child (16b) and an infant (16c) .

9. Apparatus according to one of the preceding claims, **characterized in that** it additionally comprises a validation button (19) for confirming the selection of a ventilation mode (Mode 1-6).

10. Apparatus according to one of the preceding claims, **characterized in that** the control means (4) cooperate with the storage means (8) in order to retrieve the one or more stored ventilation modes (Modes 1-6) corresponding to the patient category that has been selected by means of the one or more patient category selectors (6).

11. Apparatus according to one of the preceding claims, **characterized in that** the control means (4) cooperate with the one or more ventilation mode selectors (7) in order to:
a) authorize the selection of at least one ventilation mode (Modes 1-6) by the actuation, by the user, of one or more ventilation mode selectors (7),
and/or
b) prohibit or thwart the selection of at least one ventilation mode (Modes 1-6) by the actuation, by the user, of one or more ventilation mode selectors (7).

12. Apparatus according to one of the preceding claims, **characterized in that** the control means (4) control at least the micro-blower (2) depending on the patient category and the associated ventilation mode that have been selected by means of the one or more patient category selectors (6) and of the one or more ventilation mode selectors (7).

13. Apparatus according to one of the preceding claims, **characterized in that** the control means (4) comprise at least one microprocessor (9).

14. Apparatus according to one of the preceding claims, **characterized in that** it is configured to be, as a default, in the adult patient category and in stand-by mode.

15. Apparatus according to one of the preceding claims, **characterized in that** it is configured such that, during a ventilation, modifying the ventilation mode requires successively the actuation, by the user, of:
a) the one or more ventilation mode selectors (7) for passing from a first ventilation mode (Modes 1-6) to a desired second ventilation mode (Modes 1-6) compatible with the selected patient category, and
b) the validation button (19) for confirming the selection of said selected second ventilation mode.
